# EUROPEAN PATENT APPLICATION

(11) **EP 3 839 976 A2**
(43) Date of publication of application: **23.06.2021**
(21) Application number: 21157584.0
(22) Date of filing: 17.02.2021
(51) Int. Cl.: G16H 50/80, G16H 50/30, G16H 50/70

(54) **METHOD, APPARATUS AND DEVICE FOR PROCESSING DISEASE INFORMATION, AND STORAGE MEDIUM**

(30) Priority: 14.04.2020 CN 202010289902
(71) Applicant: BAIDU ONLINE NETWORK TECHNOLOGY (BEIJING) CO., LTD., Beijing 100085 (CN)
(72) Inventor: LIU, Junqi, Beijing, 100085 (CN)
(74) Representative: Studio Torta S.p.A.

(57) **Abstract**

The present application discloses a method, an apparatus, and a device for processing disease information and a storage medium, and relates to a big data analysis technology. A specific implementation of the method for processing disease information is: acquiring action trajectories of multiple users, where the action trajectories include location information of the multiple users at multiple time points within a preset time period; performing analysis according to disease confirmed information, a virus incubation period and the action trajectories of the multiple users, to determine at least one risk user who is at risk of infection; and pushing a notification message to each risk user, where the notification message includes disease confirmed information related to the risk user. This solution, by targeted pushing relevant information to users with higher risk based on behaviors of the users and the disease confirmed information, avoids missing important information, and the users can acquire effective information through the pushing.

## Description

### TECHNICAL FIELD

Embodiments of the present application relate to a big data analysis technology of computer technologies, in particular to a method, an apparatus, and a device for processing disease information and a storage medium.

### BACKGROUND

Recently, due to the worldwide spread of an epidemic caused by a novel coronavirus, people around the world are paying more attention to related information. Media and news report epidemic information in a form of special topics, including various types of hotspot information. Information about the epidemic basically has a strong timeliness. Various information platforms provide users with more content, which provide convenient conditions for the users to acquire information. Dependence of the users on these pages has also become important, and the users accesses these pages multiple times per day. However, for the users, there are more hotspot information updated every day, which makes them difficult to acquire effective information, and easy to miss some important related information.

### SUMMARY

A method, an apparatus and a device for processing disease information and a storage medium are provided.

According to a first aspect, a method for processing disease information is provided, which is applied to a server, where the method includes:
acquiring action trajectories of multiple users, where the action trajectories include location information of the multiple users at multiple time points within a preset time period;
performing analysis according to disease confirmed information, a virus incubation period and the action trajectories of the multiple users, to determine at least one risk user who is at risk of infection; and
pushing a notification message to each risk user, where the notification message includes disease confirmed information related to the risk user.

According to a second aspect, a method for processing disease information is provided, which is applied to a terminal device, where the method includes:
receiving a notification message sent by a server, where the notification message is determined by the server according to an action trajectory of a user, disease confirmed information, and a virus incubation period, and the notification message includes disease confirmed information related to the user; and
displaying the notification message.

According to a third aspect, an apparatus for processing disease information is provided, where the apparatus includes:
an acquiring module, configured to acquire action trajectories of multiple users, where the action trajectories include location information of the multiple users at multiple time points within a preset time period;
a processing module, configured to perform analysis according to disease confirmed information, a virus incubation period and the action trajectories of the multiple users, to determine at least one risk user who is at risk of infection; and
a pushing module, configured to push a notification message to each risk user, where the notification message includes disease confirmed information related to the risk user.

According to a fourth aspect, an apparatus for processing disease information is provided, including:
a receiving module, configured to receive a notification message sent by a server, where the notification message is determined by the server according to an action trajectory of a user, disease confirmed information, and a virus incubation period, and the notification message includes disease confirmed information related to the user; and
a displaying module, configured to display the notification message.

According to a fifth aspect, a server is provided, including:
a receiver, a processor, a transmitter, and
a memory, configured to store executable instructions;
where the processor is configured to execute the method for processing disease information according to any one of the first aspect by executing the executable instructions.

According to a sixth aspect, a terminal device is provided, including:
a receiver, a processor, a transmitter, and
a memory, configured to store executable instructions;
where the processor is configured to execute the method for processing disease information according to any one of the second aspect by executing the executable instructions.

According to a seventh aspect, a storage medium having a computer program stored thereon is provided, where the computer program implements the method for processing disease information according to the first or second aspect when the computer program is executed by a processor.

According to an eighth aspect, a method for processing disease information is provided, including:
determining at least one risk user who is at risk of infection according to disease confirmed information and action trajectories of multiple users; and
pushing a notification message to the at least one risk user.

According to a ninth aspect, a computer program product including instructions is provided, which when running on an electronic device, causes the electronic device to execute the method according to the first or second aspect.

The method, the apparatus and the device for processing disease information and the storage medium provided according to the present application, by performing analysis for each user according to behaviors of the users (that is, the action trajectories), the virus incubation period, and the confirmed information of the entire disease, to determine the user who may be at risk of infection, and pushing relevant information to this type of user, which does not need the users to manually filter, avoids missing important information, and can improve the efficiency of the users to acquire effective information.

It should be understood that the content described in this section is not intended to identify key or important features of embodiments of the present application, nor is it intended to limit the scope of the present application. Other features of the present application will be easily understood by the following description.

### BRIEF DESCRIPTION OF DRAWINGS

The attached drawings are used for a better understanding of the solutions and do not constitute a limitation of the present application. Among them:
FIG. 1 is a schematic diagram of an application scenario of a method for processing disease information according to the present application;
FIG. 2 is a schematic flowchart of Embodiment 1 of a method for processing disease information according to the present application;
FIG. 3 is a schematic flowchart of Embodiment 2 of a method for processing disease information according to the present application;
FIG. 4 is a schematic flowchart of Embodiment 3 of the method for processing disease information according to the present application;
FIG. 5 is a block diagram of Embodiment 1 of an apparatus for processing disease information according to the present application;
FIG. 6 is a block diagram of Embodiment 2 of an apparatus for processing disease information according to the present application;
FIG. 7 is a block diagram of Embodiment 3 of an apparatus for processing disease information according to the present application;
FIG. 8 is a block diagram of Embodiment 4 of an apparatus for processing disease information according to the present application;
FIG. 9 is a block diagram of Embodiment 5 of an apparatus for processing disease information according to the present application;
FIG. 10 is a block diagram of Embodiment 6 of an apparatus for processing disease information according to the present application;
FIG. 11 is a block diagram of Embodiment 7 of an apparatus for processing disease information according to the present application;
FIG. 12 is a block diagram of an embodiment of a server according to the present application; and
FIG. 13 is a block diagram of an embodiment of a terminal device according to the present application.

### DESCRIPTION OF EMBODIMENTS

Exemplary embodiments of the present application will be illustrated in combination with the accompanying drawings in the following, which include various details of the embodiments of the present application to facilitate understanding, and they should be considered as merely exemplary. Therefore, those of ordinary skill in the art should recognize that various changes and modifications can be made to the embodiments described herein without departing from the scope and spirit of the present application. Also, for clarity and conciseness, description of well-known functions and structures are omitted in the following description.

Existing news or information platforms display information on an epidemic or other diseases through special topic pages, and it is difficult for users to acquire effective information and the efficiency is low. Moreover, due to a relatively large amount of information, the users cannot acquire the effective information in time, it is also easy to miss some important related information, which is not conducive to disease prevention and control.

In view of the problems in the prior art, the present application provides a method for processing disease information, which can perform analysis and calculation on a correlation between the users and disease information according to historical behaviors of the users, and then it is more targeted when information is pushed to the users based on calculation of time and space, which does not need the users to manually filter, avoids missing important information, improves the efficiency of the users to acquire effective information, and can effectively improve the effect of pushing the disease information.

This solution may be applied to the field of treatment of infectious diseases, such as: SARS, Ebola, and diseases caused by viruses such as new coronavirus, and the like. In this way, the users can effectively learn about direct relevant information, and spreading and diffusion of the diseases are controlled.

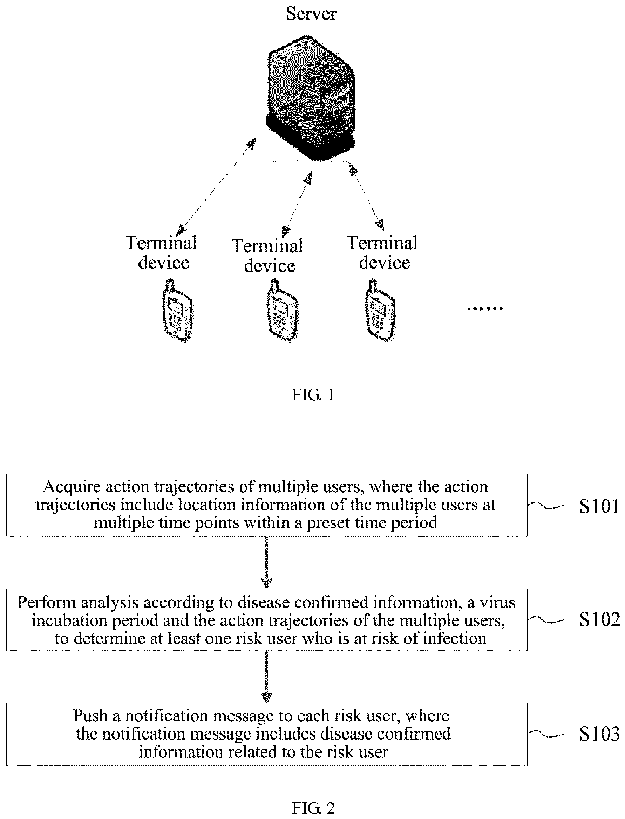

FIG. 1 is a schematic diagram of an application scenario of a method for processing disease information according to the present application. As shown in FIG. 1, a method of processing disease information provided by the present application mainly involves two devices. One is a server for users to perform data collection and analysis operation, which may be a cloud server. The other one is a terminal device on a user side. The server can acquire locations of the users at different times in time through interaction with the terminal device on the user side; and can also interact with other information platforms to acquire disease confirmed information, treatment information, rehabilitation information and the like in time. The terminal device may be mobile phone, tablet, smart watch, bracelet and other smart wearable devices of the users, which can perform data interaction with the server, and can realize a positioning function to acquire location information.

Since a specific application of this solution involves a comprehensive analysis of users in one region or across the country, the implementation of this solution involves terminal devices of multiple users, which means the amount of the terminal devices is relatively large, FIG. 1 is just an example for illustration.

FIG. 2 is a schematic flowchart of Embodiment 1 of a method for processing disease information according to the present application. As shown in FIG. 2, in a scenario shown in FIG. 1 above, on a server side, the method for processing disease information specifically includes the following steps:
S101: acquiring action trajectories of multiple users, where the action trajectories include location information of the multiple users at multiple time points within a preset time period.

In a specific implementation of this step, for the server, in order to better analyze whether a user is at a certain risk of infection, or whether a special reminder is needed, and the like, it needs to know real-time locations of the multiple users and where they have gone within a time period. In this solution, it is mainly necessary to acquire the action trajectories of the multiple users. This solution does not limit how long the action trajectories need to be acquired. The action trajectories may be within a time period, for example, may be a time period after a disease occurs, such as one month, two weeks, and the like. The length of the specific preset time period may be configured according to actual situations, for example, may be set according to an incubation period of a virus.

Preferably, an action trajectory of each user may be acquired, that is, location information of all users at each time point may be acquired.

In a specific implementation of this solution, it should be understood that each user here refers to all users within a certain area, for example, a certain cell, a certain area in a city, or a certain city, or a certain province, or the whole country. Each user may also be all users who have registered for a certain application, such as all WeChat users, all users who use netdisks or news applications. Each user may also refer to all users of a certain operator, for example, all telecommunication users, all Unicom users, all mobile users, and so on. How much user data the server can specific acquire may be set according to application scenarios of actual solutions. The core of this solution is to make more statistics of detailed information for more users, and analysis results will be more accurate.

The action trajectories of the multiple users acquired by the server must at least include the location information at each time point. These time points may be continuous, that is, the location information of the multiple users is acquired in real time. Each time point here refers to a time point at which the location information is collected within a time period, and there may also be a certain interval between time points, which may be a time of every one minute, may also be five minutes, ten minutes, fifteen minutes, half an hour, and the like. Each time the location information is collected, specific time information, that is, the time point, is acquired at the same time. It should be understood that, in the technical solutions of the present application, a time interval for collecting location information is not limited, and it may be set according to actual situations.

S102: perform analysis according to disease confirmed information, a virus incubation period and the action trajectories of the multiple users, to determine at least one risk user who is at risk of infection.

In this step, after acquiring the action trajectories of the multiple users, the server may perform analysis on situation of each user according to information such as pre-acquired disease confirmed information, virus incubation period and the like, to determine whether each user has been in contact or may have been in contact, or has been to the same place with a confirmed patient, and the like. Through this type of analysis, the at least one risk user who is at risk of infection is determined.

In a specific implementation of this solution, the disease confirmed information includes a lot of information, which may include identity information of a confirmed case and an action trajectory of the confirmed case, and may also include symptoms of the confirmed case at each stage, or may also include expert advices, protection advices, isolation advices, consultation advices, and the like, and may also include infectiousness, infection manners, and the like.

S103: push a notification message to each risk user, where the notification message includes disease confirmed information related to the risk user.

In this step, after analyzing a risk user who may be at risk of infection, the server pushes a notification message to the user by performing interaction with a terminal device of the user, so that the user can learn about the diseases confirmed information related to itself, and pay attention to personal safety, and can be timely treated if there is a risk.

In a specific implementation, the interaction between the server and the terminal device may be performed through an information platform, and communication may be performed through an application client installed on the terminal device, such as: browser, news APP, social APP, and the like; also, pushing the notification message to the terminal devices may be performed through manners such as short message or phone call, or the like by an operator; also, reminding may be performed through a system of the terminal device, and the notification message is pushed to the terminal device through a system-level message, which is not limited by this solution.

The method for processing disease information provided in this embodiment, by performing analysis for each user according to behaviors of the users (that is, the action trajectories), the virus incubation period, and the confirmed information of the entire disease, to determine the user who may be at risk of infection, and pushing relevant information to this type of user, which does not need the users to manually filter, avoids missing important information, and can improve the efficiency of the users to acquire effective information.

In a specific implementation of the above embodiment, there are many ways for the server to analyze and determine the users who are at risk of infection. Two examples are given below for illustration.

Manner 1: the disease confirmed information includes an identity and a confirmed time of a confirmed user, the perform analysis according to disease confirmed information, a virus incubation period and the action trajectories of the multiple users, to determine at least one risk user who is at risk of infection in step S102 may be implemented as:
acquire an action trajectory of the confirmed user according to the identity of the confirmed user; and determine the at least one risk user who is at risk of infection according to the virus incubation period, the confirmed time of the confirmed user, the action trajectory of the confirmed user, and action trajectories of multiple other users, wherein the at least one risk user and the confirmed user are within the same area at at least one time point in the virus incubation period.

The meaning of this solution is to find and acquire the action trajectory of the confirmed user from all action trajectories according to an action trajectory of each user over a time period acquired by the server combining with the acquired identity of the confirmed user, where the confirmed user here refers to all confirmed users.

Then, for each other user, an action trajectory of this user is compared with the action trajectories of all the confirmed users to determine whether there is a situation that the action trajectory of this user and the action trajectories of the confirmed users overlap in time and space, that is, it refers to a situation of simultaneously appearing in the same place. In this way, a user who may have been in contact with a confirmed case are screened out as a risk user from a large number of users. Or, a user who may have been in contact with a confirmed case after they are likely to be infected may be further determined as the risk users by combining a confirmed time of the confirmed case and the virus incubation period.

Manner 2: the disease confirmed information includes an identity, a confirmed time, and a time when symptoms appear of a confirmed user, and the perform analysis according to disease confirmed information, a virus incubation period and the action trajectories of the multiple users, to determine at least one risk user who is at risk of infection includes:
acquire a first action trajectory of the confirmed user in a first time period according to the identity, the confirmed time, and the time when the symptoms appear of the confirmed user, wherein the first time period comprises the virus incubation period before the symptoms appear and a time from the symptoms appear to a time when the confirmed user is confirmed and isolated; and determine the at least one risk user who is at risk of infection according to the first action trajectory of the confirmed user and action trajectories of multiple other users, wherein the at least one risk user and the confirmed user are within the same area at at least one time point in the first time period.

Different from the manner 1 above, a time period is first determined, the first time period that the confirmed case is likely to be infected is acquired, which includes a time period after being confirmed and the virus incubation period before being confirmed. According to the first time period, the first action trajectory of the confirmed user during this time period is acquired, and then a user who may have been in contact with the confirmed case in the first time period is determined as risk users by combining with the action trajectories of multiple other users, that is to say, a user who have been in contact with a certain confirmed user during an incubation stage before such user is confirmed and a time period after the symptoms appear and before such user is confirmed and isolated, or a user who has been in the same location with this confirmed user at the same time is at a certain risk of infection.

In a specific implementation, the risk user may also be accurately determined by combining with accurately positioning and officially confirmed spreading distances of the virus, such as two meters or one meter, or the like, which is not limited by this solution.

Through the above implementations, the user who is at risk of infection can be screened out by pre-acquiring the action trajectories of the multiple users, and combining the information of the confirmed case, that is, the disease confirmed information, and the notification message may be pushed to this type of user to perform a certain reminder, which avoids that user-oriented information is too broad and the amount of information is too large, making it not easy to be filtered, and solves the problem that it is difficult for the users to acquire effective information.

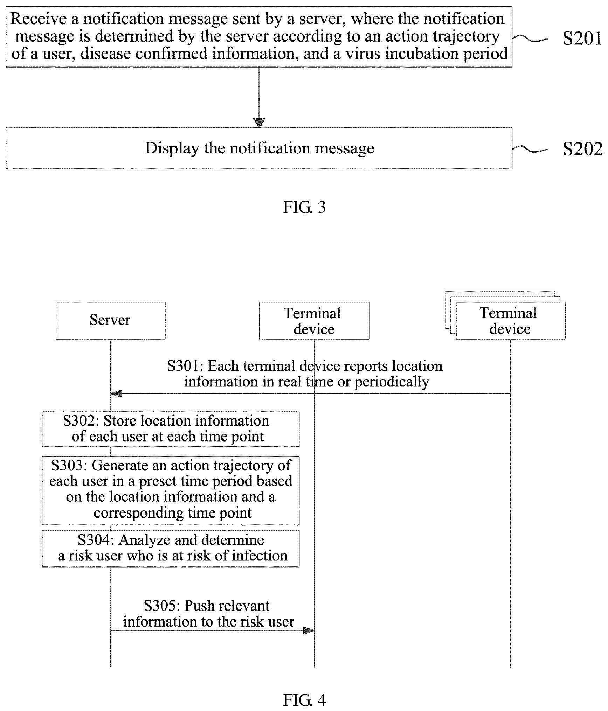

FIG. 3 is a schematic flowchart of Embodiment 2 of a method for processing disease information according to the present application; as shown in FIG. 3, in a terminal device of a user, specific implementation steps of the method for processing disease information are as follows:
S201: receive a notification message sent by a server, where the notification message is determined by the server according to an action trajectory of a user, disease confirmed information, and a virus incubation period.

In this step, for a user who is at risk of infection, the notification message includes disease confirmed information related to the user. The notification message is obtained by analyzing by the server based on the action trajectory of the user and combining with all confirmed cases and the virus incubation period, which is not information that is broad and consistent for all users.

S202: display the notification message.

In this step, after receiving the notification message, the terminal device needs to display the notification message to the user. In a specific implementation, it may be displayed in an application installed in the terminal device, or may be displayed in a short message. The notification message may also be displayed in a form of a system notification message, so that the user can acquire the disease confirmed information related to itself.

The related disease confirmed information includes information about the confirmed user who may have been contacted, as well as time and location of possible contact, a current situation of the confirmed user, may also include the action trajectory of the confirmed case, and may also include symptoms of the confirmed case at each stage, or may also include expert advices, protection advices, isolation advices, consultation advices, and the like, and may also include infectiousness, infection manners, and the like, which is not limited by this solution.

In the following, the method for processing disease information provided by the present application will be completely illustrated through a specific implementation by combining the above-mentioned embodiments.

FIG. 4 is a schematic flowchart of Embodiment 3 of a method for processing disease information according to the present application; as shown in FIG. 4, on the basis of any of the above embodiments, an implementation of this solution includes two processes, one is collection of action trajectories of multiple users, and the other one is to integrate various data for analysis. The method for processing disease information specifically includes the following steps:
S301: each terminal device reports location information in real time or periodically.

In this step, each terminal device is provided with a positioning apparatus therein, such as GPS apparatus or lidar, or the like. Each terminal device performs positioning in real-time or periodically according to the positioning apparatus provided therein, to acquire the location information of the terminal device, and sends the acquired location information to a server.

A specific reporting manner may be a manner of real-time acquiring and real-time reporting, may also be a manner of real-time acquiring and periodically reporting to the server, and may also be a manner of periodically acquiring location information according to a preset period, and then reporting to the server, which is not limited by this solution.

S302: the server stores location information of each user at each time point.

In a specific implementation of this solution, the server may store the acquired real-time location information of all users and store locally for analysis during subsequent applications.

In a specific implementation, the real-time location information may be stored for each user according to identify of the user such as mobile phone number, identify card number, name, and the like.

S303: generate an action trajectory of each user in a preset time period based on the location information and a corresponding time point.

In a specific implementation of this step, the server may generate a total action trajectory based on locations of a user at all time points, but in a specific application, especially in a process of disease prevention and control, it is mainly that an action trajectory after the disease occurs is more effective. Therefore, this step may be implemented as:
receive location information reported in real-time or periodically by terminal devices of the multiple users within the preset time period, where the preset time period is a time period after a disease original time; and generate, for each user, an action trajectory of the user in the preset time period through location information at each time point.

S304: analyze and determine a risk user who is at risk of infection.

A specific implementation of this step is similar to the manner disclosed in the foregoing embodiment. Reference is made to the foregoing solution for the description of the specific implementation in S 102.

S305: push relevant information to the risk user.

In this step, the server may push the relevant information in at least the following ways:
push the notification message through a client installed on a terminal device of each risk user; or, push the notification message by sending a system message through the terminal device of each risk user; or push the notification message to the terminal device through an operator corresponding to each risk user, and the specific manners for pushing the notification message is not limited by this solution.

The notification message includes disease confirmed information related to this user, such as information of the confirmed user, the action trajectory of the confirmed user, the virus incubation period, and the like, but also includes at least one of the following: disease defense information, disease relief information, and disease diagnosis and treatment information.

In the following, this solution is illustrated through a specific example by combining with the above-mentioned various embodiments.

The overall idea is to acquire the location information of the user in real time (or periodically) on a terminal device side and report it to the server. When the disease occurs, the server sends a notification to the user (or contacts the user) by combining geographic information of the disease. In a specific implementation of this solution, at least the following two modules must be implemented on the terminal device side:
a location information collecting module: configured to achieve collection of the location information (geographical location) of the user in real time or periodically; and
a location information reporting module: configured to report the location information of the user to a server side, that is, report to the server.

The server side needs to implement at least the following modules:
a user location information storing module: configured to store the location information of the user in a cloud;
a disease related information storing module: configured to achieve acquiring and storing of the disease confirmed information, where the stored disease confirmed information may also be for a user, and may also be for a cell; and
a disease related calculating module: configured to achieve matching of disease related location information and time information with time and space of each user; where if there is a success match, an active notification is made. That is to say, if a user appears in a cell where a confirmed user is located, or is in the same location with the confirmed user at the same time, an active notification is made.

It is assumed that, there are six cells A, B, C, D, E, F, there are 1-240 time periods, there are six people v, w, x, y, z, u, and an incubation period of the virus is 120 time periods;
It is assumed that: x lives in cell A, during a period, goes to cell C during time periods 50-80, and goes to cell D during time periods 120-160;
y lives in cell B, and goes to cell C during time periods 70-100;
z lives in cell E;
v lives in cell C;
w is a patient, and also lives in cell C; and
u lives in cell F;
then when a disease occurs in cell C at time period 110, the server needs to make the following pushing of a notification message after analysis:
a) send a notification to x and y to inform that a cell that x and y have been visited has a disease and a related situation, such as residence information, activity information, and the like;
b) notify this kind of related personnel such as v in this cell; and
c) at the same time, when another user, such as z, is going to pass the cell C later, he will be reminded to protect, or he will be reminded after he has passed the cell C.

In addition, it is assumed that user w is a patient, when a system determines a disease state of w, it will automatically calculate an intersecting time point and an action trajectory, and the like. The server needs to do the following processing:
a) for example, w has been to cell F at time period 40 (within an incubation period), and a reminder may be made; and
b) it needs to notify v that there is a case in this cell.

The method for processing disease information provided by the present application is to perform relevant calculation according to historical time and space information, and actively notify the user; during the disease time, provide the user with real-time and accurate information transmission, and actively help the user to acquire valuable information, which improves the efficiency of information acquisition. For an information pusher, it depends on the inevitability of scenarios, and ultimately actively provides the user with key information, extends search and browsing business, improves user experience and increases user stickiness at the same time.

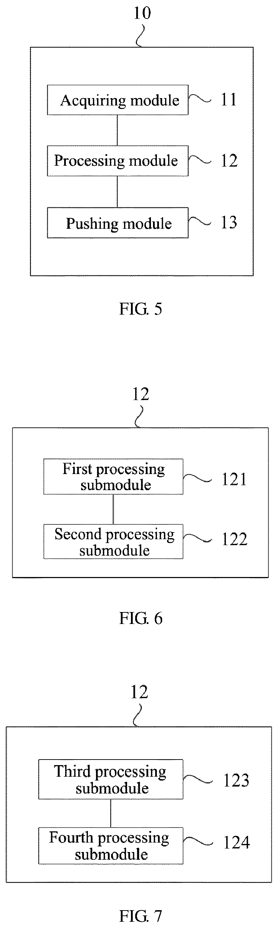

FIG. 5 is a block diagram of Embodiment 1 of an apparatus for processing disease information according to the present application. As shown in FIG. 5, the apparatus for processing disease information 10 includes:
an acquiring module 11, configured to acquire action trajectories of multiple users, where the action trajectories include location information of the multiple users at multiple time points within a preset time period;
a processing module 12, configured to perform analysis according to disease confirmed information, a virus incubation period and the action trajectories of the multiple users, to determine at least one risk user who is at risk of infection; and
a pushing module 13, configured to push a notification message to each risk user, where the notification message includes disease confirmed information related to the risk user.

FIG. 6 is a block diagram of Embodiment 2 of an apparatus for processing disease information according to the present application. As shown in FIG. 6, on the basis of the foregoing embodiment, the disease confirmed information includes an identity and a confirmed time of a confirmed user, and the processing module 12 includes:
a first processing sub-module 121, configured to an action trajectory of the confirmed user according to the identity of the confirmed user; and
a second processing sub-module 122, configured to determine the at least one risk user who is at risk of infection according to the virus incubation period, the confirmed time of the confirmed user, the action trajectory of the confirmed user, and action trajectories of multiple other users, wherein the at least one risk user and the confirmed user are within the same area at at least one time point in the virus incubation period.

FIG. 7 is a block diagram of Embodiment 3 of an apparatus for processing disease information according to the present application. As shown in FIG. 7, on the basis of the above-mentioned Embodiment 1, the disease confirmed information includes an identity, a confirmed time, and a time when symptoms appear of a confirmed user, and the processing module 12 includes:
a third processing sub-module 123, configured to acquire a first action trajectory of the confirmed user in a first time period according to the identity, the confirmed time, and the time when the symptoms appear of the confirmed user, wherein the first time period comprises the virus incubation period before the symptoms appear and a time from the symptoms appear to a time when the confirmed user is confirmed and isolated; and
a fourth processing sub-module 124, configured to determine the at least one risk user who is at risk of infection according to the first action trajectory of the confirmed user and action trajectories of multiple other users, wherein the at least one risk user and the confirmed user are within the same area at at least one time point in the first time period.

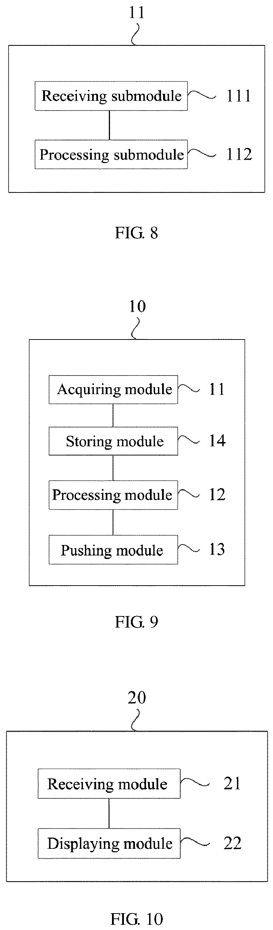

FIG. 8 is a block diagram of Embodiment 4 of an apparatus for processing disease information according to the present application. As shown in FIG. 8, on the basis of any of the above embodiments, the acquiring module 11 includes:
a receiving submodule 111, configured to receive location information reported in real-time or periodically by terminal devices of the multiple users within the preset time period, where the preset time period is a time period after a disease original time; and
a processing sub-module 112, configured to generate, for each user, an action trajectory of the user in the preset time period through location information at each time point.

FIG. 9 is a block diagram of Embodiment 5 of an apparatus for processing disease information according to the present application. As shown in FIG. 9, based on any of the above embodiments, the apparatus for processing disease information 10 further includes:
a storing module 14, configured to store the location information of each user at each time point.

In a specific implementation, on the basis of any of the foregoing embodiments, the pushing module 13 is specifically configured to:
push the notification message through a client installed on a terminal device of each risk user;
or,
push the notification message by sending a system message through the terminal device of each risk user;
or,
push the notification message to the terminal device through an operator corresponding to each risk user;
where, the notification message further includes at least one of the following: disease defense information, disease relief information, and disease diagnosis and treatment information.

The apparatus for processing disease information provided in any of the foregoing embodiments is used to implement the technical solutions on a server side in any of the foregoing method embodiments. The implementation principles and technical effects are similar, and will not be repeated here.

FIG. 10 is a block diagram of Embodiment 6 of an apparatus for processing disease information according to the present application. As shown in FIG. 10, the apparatus for processing disease information 20 includes:
a receiving module 21, configured to receive a notification message sent by a server, where the notification message is determined by the server according to an action trajectory of a user, disease confirmed information, and a virus incubation period, and the notification message includes disease confirmed information related to the user; and
a displaying module 22, configured to display the notification message.

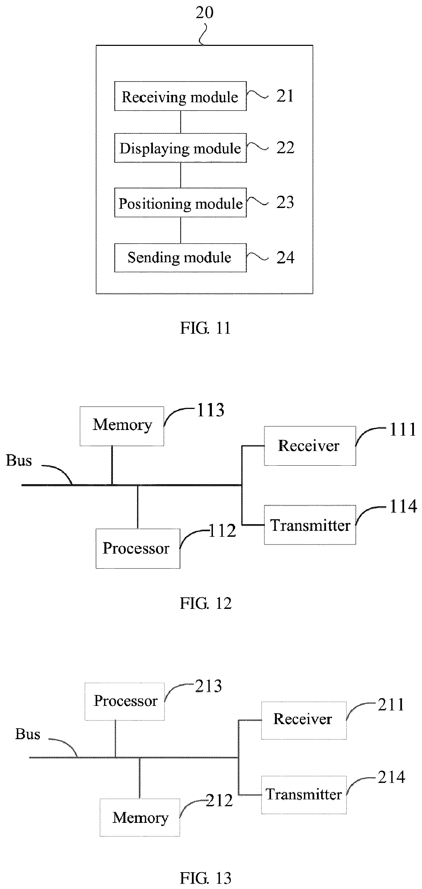

FIG. 11 is a block diagram of Embodiment 7 of an apparatus for processing disease information according to the present application. As shown in FIG. 11, based on the embodiment shown in FIG. 10, the apparatus for processing disease information 20 further includes:
a positioning module 23, configured to perform positioning in real time or periodically to acquire location information where the apparatus for processing disease information is located; and
a sending module 24, configured to send the acquired location information to the server.

The apparatuses for processing disease information provided by the above two embodiments are used to implement the technical solutions on a terminal device side in any of the foregoing method embodiments. The implementation principles and technical effects are similar, and will not be repeated here.

FIG. 12 is a block diagram of an embodiment of a server according to the present application. As shown in FIG. 12, the server includes:
a receiver 111, a processor 112, a transmitter 114, and,
the memory 113 is configured to store executable instructions;
where the processor 112 is configured to execute the method for processing disease information on a server side in any of the foregoing method embodiments by executing the executable instructions.

FIG. 13 is a block diagram of an embodiment of a terminal device according to the present application. As shown in FIG. 13, the terminal device includes:
a receiver 211, a processor 212, a transmitter 214, and,
the memory 213 is configured to store executable instructions;
where the processor 212 is configured to execute the method for processing disease information on a terminal device side in any of the foregoing method embodiments by executing the executable instructions.

A cooperation between the server and the terminal device of each user provided in the above embodiments can implement the method for processing disease information provided by the present application. Specifically, the server determines the at least one risk user who is at risk of infection according to the disease confirmed information and the action trajectories of the multiple users, and pushes the notification message to the at least one risk user.

The present application also provides a storage medium with a computer program stored thereon, and the computer program when executed by a processor, implements the technical solutions of the method for processing disease information on a server side or a terminal device side provided by any of the foregoing embodiments.

The present application also provides a computer program product containing instructions, where the instructions are stored in a readable storage medium, at least one processor of an electronic device can read the instructions from the readable storage medium, and the at least one processor executes the instructions to enable the electronic device to execute the solution according to any one of the above embodiments.

As shown in FIG. 12 and 13, which are block diagrams of a terminal device and a server that implement the method for processing disease information of the embodiments of the present application. In this solution, the terminal device generally represent various forms of mobile apparatuses, such as personal digital assistant, cellular phone, smart phone, wearable device and other similar computing apparatuses that can positioning and perform data transmission. The components shown herein, their connections and relationships, and their functions are merely examples, and are not intended to limit the implementations of the present application described and/or claimed herein.

In a specific implementation of the server or the terminal device, may include: one or more processors, a memory, and interfaces for connecting various components, including high-speed interfaces and low-speed interfaces. The various components are interconnected using different buses, and can be mounted on a common motherboard or otherwise installed as required. The processor may process instructions executed within the electronic device, including instructions stored in or on the memory to display graphical information of Graphical User Interface (GUI) on an external input/output apparatus, such as a display device coupled to an interface. In other implementations, a plurality of processors and/or a plurality of buses may be used together with a plurality of memories, if desired. Similarly, a plurality of electronic devices can be connected, and each device provides part of necessary operations (for example, as a server array, a group of blade servers, or a multi-processor system).

The memory is the non-transitory computer-readable storage medium provided by the present application. The memory stores instructions executable by at least one processor, so that the at least one processor executes the method for processing disease information provided by the present application.

The non-transitory computer-readable storage medium of the present application stores computer instructions, the computer instructions are used to cause a computer to execute the method for processing disease information provided by the present application.

As a non-transitory computer-readable storage medium, the memory may be configured to store non-transitory software programs, non-transitory computer-executable programs and modules, such as program instructions/modules corresponding to the method for processing disease information in the embodiments of the present application. In the same device, the processor executes various functional applications and data processing of the server by running non-transitory software programs, instructions and modules stored in the memory, that is, the method for processing disease information in the above-mentioned method embodiments are realized.

The memory may include a storage program area and a storage data area, where the storage program area may store an operating system and at least one application program required for functions; the storage data area may store data created according to the use of the electronic device of the method for processing disease information, and the like. In addition, the memory may include high-speed random access memory, and may also include non-transitory memory, such as at least one magnetic disk memory device, flash memory device, or other non-transitory solid-state memory devices. In some embodiments, the memory may include memories remotely disposed with respect to the processor, and these remote memories may be connected to the server through a network. Examples of the above-mentioned network include, but are not limited to, an Internet, an intranet, a local area network, a mobile communication network and combination thereof.

In addition, the aforementioned server or terminal device may also include: a receiver or a transmitter, which may also be referred to as an input apparatus or an output apparatus. The processor, the memory, the receiver, and the transmitter may be connected through a bus or in other manners. In the above embodiments, a connection through a bus is taken as an example.

The receiver (or input apparatus) may receive input numeric or character information, and generate key signal inputs related to user settings and function control, for example input apparatus such as touch screen, keypad, mouse, track pad, touch pad, pointing stick, one or more mouse buttons, trackball, joystick. The output apparatus may include a display device, an auxiliary lighting apparatus (e.g., an LED), a haptic feedback apparatus (e.g., a vibration motor), and the like. The display device may include, but is not limited to, a liquid crystal display (LCD), a light-emitting diode (LED) display, and a plasma display. In some implementations, the display device may be a touch screen.

Various implementations of the systems and technologies described herein may be implemented in a digital electronic circuit system, an integrated circuit system, an application-specific ASIC (application-specific integrated circuit), computer hardware, firmware, software, and/or combination thereof. These various implementations may include: implemented in one or more computer programs, the one or more computer programs are executable and/or interpreted on a programmable system including at least one programmable processor, the programmable processor may be a dedicated or general-purpose programmable processor that may receive data and instructions from a storage system, at least one input device, and at least one output device, and transmit data and instructions to the storage system, at least one input apparatus, and at least one output apparatus.

These computer programs (also known as programs, software, software applications, or codes) include machine instructions of a programmable processor, and can be implemented by using high-level procedures and/or object-oriented programming languages, and/or assembly/machine languages. As used herein, the terms "machine-readable medium" and "computer-readable medium" refer to any computer program product, device, and/or apparatus used to provide machine instructions and/or data to a programmable processor (e.g., a magnetic disk, an optical disk, a memory, a programmable logic device (PLD)), including machine-readable medium that receive machine instructions as machine-readable signals. The term "machine-readable signal" refers to any signal used to provide machine instructions and/or data to a programmable processor.

To provide interaction with the user, the systems and technologies described herein can be implemented on a computer having: a display apparatus (e.g., a CRT (cathode ray tube) or LCD (liquid crystal display) monitor) for displaying information to the user; and a keyboard and pointing device (e.g., a mouse or a trackball) through which the user can provide input to the computer. Other kinds of apparatuses may also be used to provide interaction with a user; for example, the feedback provided to the user may be any form of sensor feedback (for example, visual feedback, audible feedback, or haptic feedback); and may receive input from the user in any form, including acoustic input, voice input or haptic input.

The systems and technologies described herein can be implemented in a computing system including background components (e.g., as a data server), or a computing system including middleware components (e.g., an application server), or a computing system including front-end components (e.g., a user computer with a graphical user interface or a web browser through which users can interact with implementation of the systems and technologies described herein), or a computing system that includes any combination of such back-end components, middleware components, or front-end components. The components of the systems can be interconnected by any form or medium of digital data communication of digital data communication (e.g., a communication network). Examples of communication networks include local area network (LAN), wide area network (WAN), and Internet.

The computing system may include a client side and a server. The client side and the server are generally remote from each other and typically interact through a communication network. The relationship between the client side and server is generated by computer programs running on a corresponding computer and having a client side-server relationship with each other.

It should be understood that various forms of processes shown above can be used to reorder, add, or delete steps. For example, various steps recorded in the present application can be executed in parallel, sequentially or in different orders. As long as the desired results of the technical solutions disclosed in the present application can be achieved, there is no limitation herein.

The above-mentioned specific implementations do not constitute a limitation of the protection scope of the present application. It should be understood by those skilled in the art that various modifications, combinations, sub-combinations and substitutions may be made according to design requirements and other factors. Any modification, equivalent replacement, improvement and the like made within the spirit and principle of the present application shall be included in the protection scope of the present application.

## Claims

1. A method for processing disease information, which is applied to a server, wherein the method comprises:
acquiring (S101) action trajectories of multiple users, wherein the action trajectories comprise location information of the multiple users at multiple time points within a preset time period;
performing (S102) analysis according to disease confirmed information, a virus incubation period and the action trajectories of the multiple users, to determine at least one risk user who is at risk of infection; and
pushing (S103) a notification message to each risk user, wherein the notification message comprises disease confirmed information related to the risk user.

2. The method according to claim 1, wherein the disease confirmed information comprises an identity and a confirmed time of a confirmed user, and the performing (S102) analysis according to disease confirmed information, a virus incubation period and the action trajectories of the multiple users, to determine at least one risk user who is at risk of infection comprises:
acquiring an action trajectory of the confirmed user according to the identity of the confirmed user; and
determining the at least one risk user who is at risk of infection according to the virus incubation period, the confirmed time of the confirmed user, the action trajectory of the confirmed user, and action trajectories of multiple other users, wherein the at least one risk user and the confirmed user are within the same area at at least one time point in the virus incubation period.

3. The method according to claim 1, wherein the disease confirmed information comprises an identity, a confirmed time, and a time when symptoms appear of a confirmed user, and the performing (S102) analysis according to disease confirmed information, a virus incubation period and the action trajectories of the multiple users, to determine at least one risk user who is at risk of infection comprises:
acquiring a first action trajectory of the confirmed user in a first time period according to the identity, the confirmed time, and the time when the symptoms appear of the confirmed user, wherein the first time period comprises the virus incubation period before the symptoms appear and a time from the symptoms appear to a time when the confirmed user is confirmed and isolated; and
determining the at least one risk user who is at risk of infection according to the first action trajectory of the confirmed user and action trajectories of multiple other users, wherein the at least one risk user and the confirmed user are within the same area at at least one time point in the first time period.

4. The method according to any one of claims 1 to 3, wherein the acquiring (S101) action trajectories of multiple users comprises:
receiving location information reported in real-time or periodically by terminal devices of the multiple users within the preset time period, wherein the preset time period is a time period after a disease original time; and
generating (S303), for each user, an action trajectory of the user in the preset time period through location information at each time point.

5. The method according to claim 4, further comprising:
storing (S302) the location information of each user at each time point.

6. The method according to any one of claims 1 to 3, wherein the pushing (S103) a notification message to each risk user comprises:
pushing the notification message through a client installed on a terminal device of each risk user;
or,
pushing the notification message by sending a system message through the terminal device of each risk user;
or,
pushing the notification message to the terminal device through an operator corresponding to each risk user;
wherein, the notification message further comprises at least one of the following: disease defense information, disease relief information, and disease diagnosis and treatment information.

7. A method for processing disease information, which is applied to a terminal device, wherein the method comprises:
receiving (S201) a notification message sent by a server, wherein the notification message is determined by the server according to an action trajectory of a user, disease confirmed information, and a virus incubation period, and the notification message comprises disease confirmed information related to the user; and
displaying (S202) the notification message.

8. The method according to claim 7, further comprising:
performing positioning in real time or periodically according to a positioning apparatus provided in the terminal device, to acquire location information where the terminal device is located, and sending the acquired location information to the server.

9. An apparatus for processing disease information, comprising:
an acquiring module (11), configured to acquire action trajectories of multiple users, wherein the action trajectories comprise location information of the multiple users at multiple time points within a preset time period;
a processing module (12), configured to perform analysis according to disease confirmed information, a virus incubation period and the action trajectories of the multiple users, to determine at least one risk user who is at risk of infection; and
a pushing module (13), configured to push a notification message to each risk user, wherein the notification message comprises disease confirmed information related to the risk user.

10. An apparatus for processing disease information, comprising:
a receiving module (21), configured to receive a notification message sent by a server, wherein the notification message is determined by the server according to an action trajectory of a user, disease confirmed information, and a virus incubation period, and the notification message comprises disease confirmed information related to the user; and
a displaying module (22), configured to display the notification message.

11. A server, comprising:
a receiver (111), a processor (112), a transmitter (114), and
a memory (113), configured to store executable instructions;
wherein the processor (112) is configured to execute the method for processing disease information according to any one of claims 1 to 6 by executing the executable instructions.

12. A terminal device, comprising:
a receiver (211), a processor (212), a transmitter (214), and
a memory (213), configured to store executable instructions;
wherein the processor (212) is configured to execute the method for processing disease information according to claim 7 or 8 by executing the executable instructions.

13. A computer program product comprising instructions, which when running on an electronic device, causes the electronic device to execute the method according to any one of claims 1 to 8.

14. A storage medium having a computer program stored thereon, wherein the computer program implements the method for processing disease information according to any one of claims 1 to 8 when the computer program is executed by a processor.

15. A method for processing disease information, comprising:
determining at least one risk user who is at risk of infection according to disease confirmed information and action trajectories of multiple users; and
pushing a notification message to the at least one risk user.
